# FASCICULE DE BREVET EUROPEEN

(11) **EP 0 705 112 B1**
(45) Date de publication et mention de la délivrance du brevet: **07.01.1998**
(21) Numéro de dépôt: 94920502.5
(22) Date de dépôt: 22.06.1994
(51) Int. Cl.: A61L 9/01

(54) **COMPOSITIONS DE DESODORISATION DES DEJECTIONS ANIMALES ET PROCEDE DE DESODORISATION CORRESPONDANT**
MISCHUNGEN ZUR DESODORISIERUNG VON TIEREXCREMENTEN UND ENTSPRECHENDES VERFAHREN ZUR DESODORISIERUNG
COMPOSITIONS FOR DEODORISING ANIMAL EXCRETA, AND DEODORISING METHOD THEREFOR

(30) Priorité: 23.06.1993 FR 9307631
(43) Date de publication de la demande: 10.04.1996
(73) Titulaire: ELF ATOCHEM S.A., 92800 Puteaux (FR)
(72) Inventeur: BASSET, Jacques, F-76410 Saint-Aubin-les-Elbeuf (FR); CAUPIN, Henri-Jean, F-78000 Versailles (FR); WABLE, Francis, F-92500 Reuil-Malmaison (FR)
(74) Mandataire: Chaillot, Geneviève
(86) Numéro de dépôt international: FR9400755
(87) Numéro de publication internationale: WO9500182

## Description

La présente invention porte sur des compositions pour la désodorisation des dejections animales nauséabondes, comme les lisiers, en particulier les lisiers de porc, et les déjections provenant des élevages de volailles : canards, poulets, etc. La présente invention porte également sur le procédé de désodorisation correspondant.

L'élevage intensif est une source importante de nuisances, le secteur des élevages porcins, avec les lisiers, en étant une composante non négligeable. Le cheptel porcin génère des quantités importantes de lisier, lesquelles, outre le problème de pollution occasionné notamment par le surépandage, sont des sources de nuisances par les odeurs, tant au niveau des bâtiments d'élevage, avec les stockages sous caillebotis, qu'au niveau de leur stockage, de leur traitement et de leur épandage.

Plusieurs systèmes de désodorisation sont connus. Ces systèmes ont pour vocation de réduire l'odeur, mais aussi les composants polluants. Souvent très onéreux, parce que nécessitant des investissements considérés bien souvent comme non-productifs, ces systèmes ne règlent pas spécifiquement, et pas toujours efficacement, le problème des odeurs.

De nombreuses substances ont été proposées pour apporter une solution aux problèmes d'odeurs de déjections animales ; elles interviennent le plus souvent comme masque d'odeur, c'est-à-dire par substitution des odeurs des déjections par celles qu'elles dégagent.

On connaît cependant, par la demande de brevet européen EP-A-0 434 523, l'emploi d'esters d'acide undécylénique polyoxyalkylénés à 2-10 motifs polyoxyalkylène, lesquels ont l'avantage de ne pas conduire à une forte perception de leur propre odeur, comme c'est le cas avec l'undécylénate de méthyle. Toutefois, il est apparu que ces agents désodorisants pour lisier devaient encore être améliorés. C'est dans cet objectif qu'a été réalisée la présente invention.

Il a en effet été découvert qu'une composition, comprenant au moins deux constituants parmi (a) les superphosphates simples, (b) l'acide undécylénique et ses esters dont l'undécylénate de méthyle, et (c) le perborate de sodium, manifestait toujours, si l'on examine les diagrammes effet/dose (en pourcentage d'intensité d'odeur) un effet de potentialisation, notamment de l'effet du superphosphate simple respectivement par l'acide ou ester undécylénique et le perborate de sodium, et du perborate de sodium par l'acide ou ester undécylénique.

Un autre effet inattendu est que l'on a pu parvenir à d'excellents résultats, par exemple à 15% d'intensité d'odeur dans le cas d'une composition formulée pour pouvoir traiter les déjections par 5000 ppm de superphosphate simple et 320 ppm d'undécylénate de méthyle.

Encore un autre effet inattendu est observé dans le fait que l'undécylénate de méthyle n'a plus son odeur propre fortement perçue lorsqu'il est au moment de l'emploi, incorporé dans les déjections en combinaison avec le superphosphate simple ou avec le superphosphate simple + perborate de sodium, le mélange des constituants n'étant effectué qu'au moment de l'emploi ou encore l'undécylénate de méthyle étant incorporé dans la masse à traiter de façon séparée des autres constituants. Dans le classement des formulations par qualité (voir Tableau 3 ci-après), si l'on compare les formulations 7b, 4b et 2b, qui permettent de traiter les déjections par 100 ppm d'undécylénate de méthyle, on ne trouve pas le même pourcentage comme l'on pouvait s'y attendre. Il est en effet bien indiqué dans la demande de brevet européen citée en référence que l'undécylénate de méthyle a une odeur propre fortement perçue.

Par ailleurs, les compositions renfermant les trois constituants fournissent d'excellents résultats, permettant notamment d'atteindre des valeurs de l'ordre de 10% de l'intensité d'odeur initiale, comme par exemple avec une composition formulée pour pouvoir traiter les déjections par 7500 ppm de superphosphate simple, 150 ppm d'undécylénate de méthyle et 300 ppm en O₂ actif de perborate de sodium.

La présente invention a donc d'abord pour objet des compositions pour la désodorisation des déjections animales comme les lisiers, en particulier les lisiers de porc, caractérisées par le fait qu'elles comprennent au moins deux constituants parmi :
(a) au moins un superphosphate simple ;
(b) au moins un composé choisi parmi l'acide undécylénique, ses esters et ses esters polyoxyalkylénés.

Les superphosphates simples sont des composés bien connus comme engrais ; ils sont constitués essentiellement par des mélanges des trois phosphates de calcium avec de l'acide phosphorique et du sulfate de calcium. On en distingue diverses variétés, à savoir les superphosphates minéraux, obtenus en partant des phosphates minéraux ; les superphosphates d'os, obtenus à partir d'os dégraissés et concassés ; les superphosphates d'ammoniaque, mélanges de sulfate d'ammonium et de superphosphate de calcium ; les superphosphates ammoniaco-nitriques, mélanges de nitrate de sodium, de sulfate d'ammonium et de superphosphate ; les superphosphates ammoniaco-potassiques, mélanges de sulfate d'ammonium, de superphosphate et de sels de potassium ; et les superphosphates ammoniaco-magnésiens, obtenus par action du phosphate de magnésium sur une solution ammoniacale.

Comme exemple de superphosphate utilisable, on peut citer un superphosphate simple à environ 16-18% en poids de P₂O₅.

Parmi les esters de l'acide undécylénique entrant dans la définition du constituant (b) de la composition conforme à la présente invention, on peut citer les undécylénates d'alkyle, comme l'undécylénate de méthyle, et les esters polyoxyalkylénes, renfermant par exemple 2 à 12 motifs oxyalkylène, comme les esters polyoxyéthylénés, polyoxypropylénés ou polyoxyéthylénés-polyoxypropylénés.

Les compositions selon l'invention peuvent également comprendre, en plus des constituants (a) et (b), du perborate de sodium (constituant (c)).

Ces compositions sont avantageusement formulées pour pouvoir traiter les déjections animales par :
- jusqu'à 10 000 ppm environ du constituant (a) ;
- jusqu'à 1000 ppm environ du constituant (b) ; et
- le cas échéant, jusqu'à 500 ppm environ en O₂ actif du constituant (c),
ces quantités étant calculées par rapport au poids des déjections animales à traiter.

De façon préférée, de telles compositions sont formulées pour pouvoir traiter les déjections animales par :
- environ 1000 à 5000 ppm du constituant (a) ;
- environ 50 à 100 ppm du constituant (b) ; et
- le cas échéant, jusqu'à 300 ppm environ en O₂ actif du constituant (c),
ces quantités étant calculées par rapport au poids des déjections animales à traiter.

De préférence, le constituant (c) est alors présent dans une quantité permettant de traiter les déjections animales par environ 150 à 300 ppm en O₂ actif.

On peut également envisager qu'une composition désodorisante selon l'invention comprenne en outre au moins un additif choisi parmi les fongicides et les agents bactériostatiques, additifs bien connus de l'homme du métier.

Les constituants d'une composition désodorisante selon l'invention peuvent être conditionnés de façon séparée avant emploi. Par ailleurs, chacun des constituants peut se présenter sous une forme pulvérulente, ou bien en suspension ou en solution dans l'eau, ou bien au moins partiellement à l'état adsorbé sur un support poreux et inerte, comme des particules d'argile ou de silice.

La présente invention a également pour objet un procédé de désodorisation des déjections animales, en particulier le lisier de porc, caractérisé par le fait que l'on incorpore par mélange aux déjections animales à traiter, en vue de leur stockage ou de leur épandage, une composition désodorisante telle que définie ci-dessus.

On ne peut préparer le mélange des constituants qu'au moment de l'emploi ou bien on peut incorporer aux déjections animales à traiter au moins un constituant de la composition de façon séparée des autres constituants.

Pour mieux illustrer l'objet de la présente invention, on va en décrire ci-après, à titre indicatif et non limitatif, plusieurs exemples de réalisation.

### Exemples de formulation 1 à 7

On a envisagé les formulations 1 à 7 de l'invention, telles que définies dans le Tableau 1, permettant de traiter le lisier par A ppm de superphosphate simple, B ppm d'undécylénate de méthyle et C ppm en O₂ actif de perborate de sodium. Le superphosphate simple utilisé était celui commercialisé par la Société SOFERTI sous la dénomination commerciale BIOSUPER.

**Tableau 1**

| Exemple de formulation | A | B | C |
|---|---|---|---|
| 1 | 1000 | 50 | 300 |
| 2 | 1000 | 100 | 0 |
| 3 | 2000 | 50 | 300 |
| 4 | 2000 | 100 | 0 |
| 5 | 2000 | 50 | 150 |
| 6 | 3000 | 50 | 150 |
| 7 | 5000 | 100 | 300 |

Chacune de ces formulations a été mise en oeuvre comme suit :

On a dilué un mélange de superphosphate simple et d'undécylénate de méthyle dans un fût à ouverture totale d'une capacité de 200 l, dans environ son poids d'eau. Le cas échéant, on a dilué le perborate de sodium dans un autre fût et dans les mêmes conditions. On a rempli une tonne à lisier, ayant une capacité de 15 000 l et munie d'un système de mélange par homogénéisation pneumatique, par pompage par aspiration simultanée de lisier et du diluat de superphosphate simple et d'undécylénate de méthyle jusqu'à la moitié du volume de la tonne. Ensuite, on a aspiré dans la tonne à lisier le diluat de perborate de sodium, s'il y avait lieu, et le reste du lisier.

Par ailleurs, deux séries d'essais ont été conduites, l'une (série a) avec un mélange superphosphate simple et undécylénate formé avant l'emploi, et l'autre (série b) avec un tel mélange réalisé juste avant l'emploi.

L'évaluation de chacune de ces formulations 1a à 7a et 1b à 7b sur l'odeur du lisier de porc à l'épandage a été effectuée par rapport à du lisier non traité comme témoin. A cet effet, le lisier témoin et les différents lisiers désodorisés conformément à l'invention ont été mis en oeuvre en plein champ en août-septembre à raison d'une dose d'épandage de 30 m³/ha. Le lisier utilisé avait un taux en matières sèches de 4 à 8% en poids et contenait 4,9% en poids d'azote déterminé par la méthode de Kjeldahl, 3,9% de phosphore calculé en poids de P₂O₅ et 2,2% de potassium calculé en poids de K₂O. Chaque essai a fait l'objet de 3 répétitions unitaires, et l'évaluation de l'efficacité a été réalisée à l'aide d'un panel d'évaluation constitué de 8 personnes. Le panel a été étalonné par olfactométrie sur du lisier et de l'ammoniaque. Les trois composantes de l'efficacité étaient les suivantes:
- Acceptabilité: : degré d'appréciation de l'odeur résiduelle, si elle existe, du lisier traité (insupportable, désagréable, acceptable) ;
- Intensité de l'odeur: : degré de perception (imperceptible, décelable à insoutenable) ;
- Qualité de l'odeur: : représentativité de l'odeur après traitement par rapport à l'odeur de lisier non traité (odeur représentative, dénaturée ou différente).

Les Tableaux 2 à 4 suivants présentent le classement des formulations selon le pourcentage de réponse respectivement de :
- - leur acceptabilité :: % de réponses "acceptable" ;
- - leur intensité :: % des réponses "imperceptible" ou "juste décelable" ;
- - leur qualité :: % des réponses "représentative du lisier".

**Tableau 2**

| Classement des formulations par leur acceptabilité (de la meilleure à la moins bonne) | |
|---|---|
| Formulation N° | % |
| 7b | 100 |
| 4b | 100 |
| 5a | 80 |
| 2b | 78 |
| 6b | 75 |
| 2a | 70 |
| 1a | 68 |
| 7a | 56 |
| 3a | 50 |
| 4a | 30 |
| 6a | 29 |

**Tableau 3**

| Classement des formulations par leur qualité (de la meilleure à la moins bonne) | |
|---|---|
| Formulation N° | % |
| 7b | 0 |
| 4b | 0 |
| 5a | 0 |
| 1a | 0 |
| 3a | 0 |
| 2a | 10 |
| 2b | 25 |
| 6b | 25 |
| 6a | 33 |
| 4a | 63 |
| 7a | 67 |

**Tableau 4**

| Classement des formulations par leur intensité (de la meilleure à la moins bonne) | |
|---|---|
| Formulation N° | % |
| 7b | 100 |
| 1a | 67 |
| 5a | 60 |
| 7a | 45 |
| 4b | 33 |
| 2a | 33 |
| 4a | 20 |
| 3a | 17 |
| 6a | 14 |
| 2a | 11 |
| 6b | 0 |

Le Tableau 5 présente le classement d'ensemble.

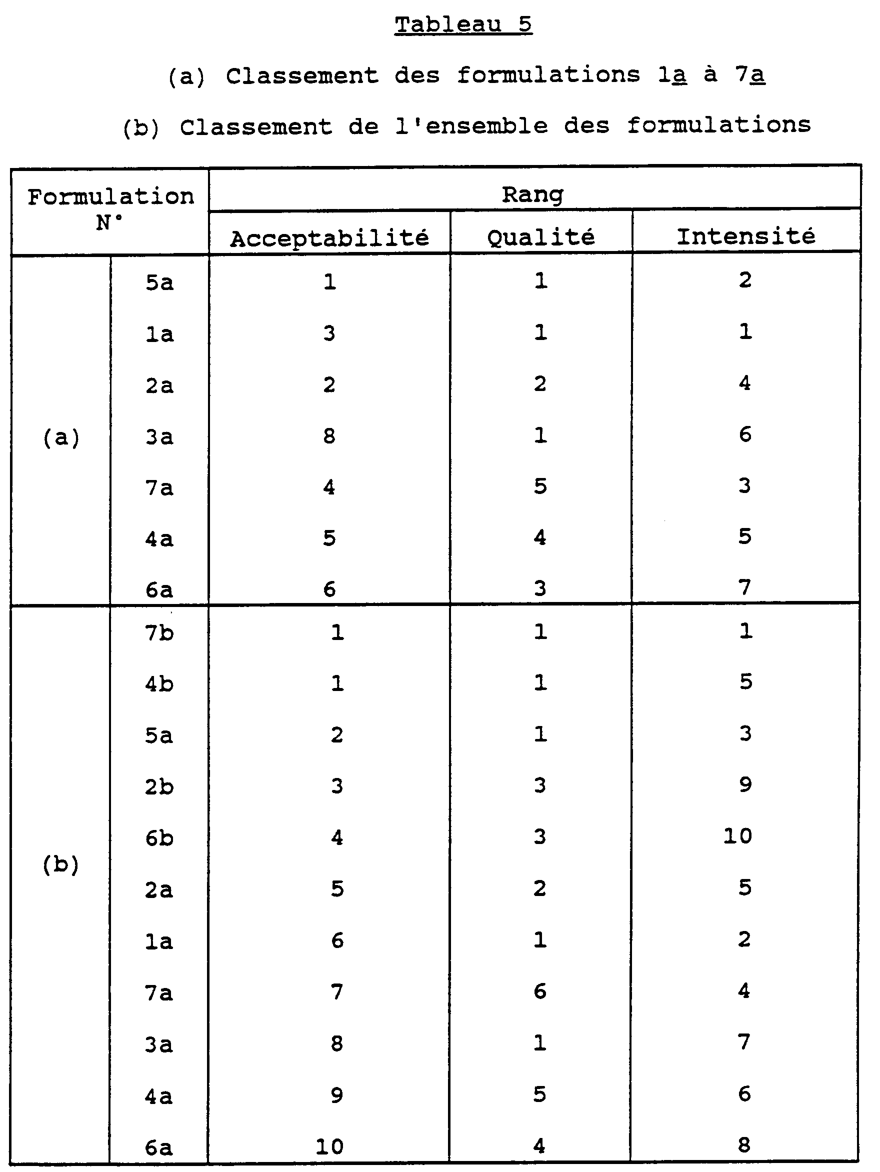

L'homme du métier sera à même de formuler les compositions désodorisantes des déjections animales pour chaque application particulière en fonction des critères sélectionnés et des coûts.

## Revendications

1. Composition pour la désodorisation des déjections animales telles que les lisiers, en particulier les lisiers de porc, caractérisée par le fait qu'elle comprend :
(a) au moins un superphosphate simple ; et
(b) au moins un composé choisi parmi l'acide undécylénique, ses esters et ses esters polyoxyalkylénés.

2. Composition selon la revendication 1, caractérisée par le fait que le constituant (a) est un superphosphate simple à environ 16-18% en poids de P₂O₅.

3. Composition selon l'une des revendications 1 et 2, caractérisée par le fait que le constituant (b) est l'undécylénate de méthyle.

4. Composition selon l'une des revendications 1 à 3, caractérisée par le fait qu'elle comprend en outre (c) du perborate de sodium.

5. Composition désodorisante selon l'une des revendication 1 à 4, caractérisée par le fait qu'elle est formulée pour pouvoir traiter les déjections animales par :
- jusqu'à 10 000 ppm environ du constituant (a) ;
- jusqu'à 1000 ppm environ du constituant (b) ; et
- le cas échéant, jusqu'à 500 ppm environ en O₂ actif du constituant (c),
ces quantités étant calculées par rapport au poids des déjections animales à traiter.

6. Composition désodorisante selon la revendication 5, caractérisée par le fait qu'elle est formulée pour pouvoir traiter les déjections animales par :
- environ 1000 à 5000 ppm du constituant (a) ;
- environ 50 à 100 ppm du constituant (b) ; et
- le cas échéant, jusqu'à 300 ppm environ en O₂ actif du constituant (c),
ces quantités étant calculées par rapport au poids des déjections animales à traiter.

7. Composition désodorisante selon les revendications 4 et 6 prises simultanément, caractérisée par le fait que le constituant (c) est présent dans une quantité permettant de traiter les déjections animales par environ 150 à 300 ppm en O₂ actif.

8. Composition désodorisante selon l'une des revendications 1 à 7, caractérisée par le fait qu'elle comprend en outre au moins un additif choisi parmi les fongicides et les agents bactériostatiques.

9. Composition désodorisante selon l'une des revendications 1 à 8, caractérisée par le fait que ses constituants sont conditionnés de façon séparée avant emploi.

10. Composition désodorisante selon l'une des revendications 1 à 9, caractérisée par le fait que chacun de ses constituants se présente sous une forme pulvérulente, ou bien en suspension ou en solution dans l'eau, ou bien au moins partiellement à l'état adsorbé sur un support poreux et inerte, comme des particules d'argile ou de silice.

11. Procédé de désodorisation des lisiers, en particulier des lisiers de porc, caractérisé par le fait que l'on incorpore par mélange aux déjections animales à traiter, en vue de leur stockage ou de leur épandage, une composition telle que définie à l'une des revendications 1 à 10.

12. Procédé selon la revendication 11, caractérisé par le fait qu'on ne prépare le mélange des constituants de la composition qu'au moment de l'emploi.

13. Procédé selon la revendication 11, caractérisé par le fait qu'au moment de l'emploi, on incorpore aux déjections animales à traiter au moins un constituant de la composition de façon séparée des autres constituants.

## Claims

1. Composition for deodorizing animal faeces such as liquid manure, in particular liquid pig manure, characterized in that it comprises:
(a) at least one normal superphosphate; and
(b) at least one compound chosen from undecylenic acid, its esters and its polyoxyalkylenated esters.

2. Composition according to Claim 1, characterized in that the constituent (a) is a normal superphosphate containing approximately 16-18% by weight of P₂O₅.

3. Composition according to either of Claims 1 and 2, characterized in that the constituent (b) is methyl undecylenate.

4. Composition according to one of Claims 1 to 3, characterized in that it additionally comprises (c) sodium perborate.

5. Deodorizing composition according to one of Claims 1 to 4, characterized in that it is formulated in order to be able to treat animal faeces with:
- up to approximately 10,000 ppm of the constituent (a);
- up to approximately 1000 ppm of the constituent (b); and
- if appropriate, up to approximately 500 ppm of active O₂ from the constituent (c),
these amounts being calculated with respect to the weight of the animal faeces to be treated.

6. Deodorizing composition according to Claim 5, characterized in that it is formulated in order to be able to treat animal faeces with:
- approximately 1000 to 5000 ppm of the constituent (a);
- approximately 50 to 100 ppm of the constituent (b); and
- if appropriate, up to approximately 300 ppm of active O₂ from the constituent (c),
these amounts being calculated with respect to the weight of the animal faeces to be treated.

7. Deodorizing composition according to Claims 4 and 6 taken simultaneously, characterized in that the constituent (c) is present in an amount which makes it possible to treat the animal faeces with approximately 150 to 300 ppm of active O₂.

8. Deodorizing composition according to one of Claims 1 to 7, characterized in that it additionally comprises at least one additive chosen from fungicides and bacteriostatic agents.

9. Deodorizing composition according to one of Claims 1 to 8, characterized in that its constituents are packaged separately before use.

10. Deodorizing composition according to one of Claims 1 to 9, characterized in that each of its constituents is provided in a pulverulent form, or else in suspension or in solution in water, or else at least partially in the form adsorbed on a porous and inert carrier such as silica or clay particles.

11. Process for deodorizing liquid manure, in particular liquid pig manure, characterized in that a composition as defined in one of Claims 1 to 10 is incorporated, by mixing, in the animal faeces to be treated, with a view to storing or spreading them.

12. Process according to Claim 11, characterized in that the mixture of the constituents of the composition is prepared only at the time of use.

13. Process according to Claim 11, characterized in that, at the time of use, at least one constituent of the composition is incorporated, separately from the other constituents, in the animal faeces to be treated.

## Patentansprüche

1. Zusammensetzung zur Beseitigung des Geruchs von tierischen Exkrementen wie Gülle, insbesondere Schweinegülle, dadurch gekennzeichnet, daß die Zusammensetzung
(a) mindestens ein einfaches Superphosphat; und
(b) mindestens eine Verbindung ausgewählt aus Undecylensäure, ihren Estern und ihren Polyoxyalkylenestern,
enthält.

2. Zusammensetzung nach Anspruch 1, dadurch gekennzeichnet, daß der Bestandteil (a) ein einfaches Superphosphat mit etwa 16 bis 18 Gew.% P₂O₅ ist.

3. Zusammensetzung nach einem der Ansprüche 1 und 2, dadurch gekennzeichnet, daß der Bestandteil (b) Methylundecylenat ist.

4. Zusammensetzung nach einem der Ansprüche 1 bis 3, dadurch gekennzeichnet, daß sie außerdem als Bestandteil (c) Natriumperborat enthält.

5. Geruchsbeseitigende Zusammensetzung nach einem der Ansprüche 1 bis 4 dadurch gekennzeichnet, daß sie zur Behandlung der tierischen Exkremente zusammengesetzt ist aus:
- bis zu etwa 10.000 ppm des Bestandteils (a);
- bis zu etwa 1.000 ppm des Bestandteils (b); und
- gegebenenfalls bis zu etwa 500 ppm an aktivem O₂ des Bestandteils (c),
wobei diese Mengen bezogen auf das Gewicht der zu behandelnden tierischen Exkremente berechnet sind.

6. Geruchsbeseitigende Zusammensetzung nach Anspruch 5, dadurch gekennzeichnet, daß sie zur Behandlung der tierischen Exkremente zusammengesetzt ist aus:
- etwa 1.000 bis 5.000 ppm des Bestandteils (a);
- etwa 50 bis 100 ppm des Bestandteils (b); und
- gegebenenfalls bis zu etwa 300 ppm an aktivem Sauerstoff des Bestandteils (c),
wobei diese Mengen bezogen auf das Gewicht der zu behandelnden tierischen Exkremente berechnet sind.

7. Geruchsbeseitigende Zusammensetzung nach den Ansprüchen 4 und 6 gleichzeitig, dadurch gekennzeichnet, daß der Bestandteil (c) in einer Menge vorhanden ist, die die Behandlung der tierischen Exkremente mit etwa 150 bis 300 ppm aktivem Sauerstoff ermöglicht.

8. Geruchsbeseitigende Zusammensetzung nach einem der Ansprüche 1 bis 7, dadurch gekennzeichnet, daß sie außerdem mindestens ein Additiv umfaßt, ausgewählt aus Fungiziden und bakterienhemmenden Reagenzien.

9. Geruchsbeseitigende Zusammensetzung nach einem der Ansprüche 1 bis 8, dadurch gekennzeichnet, daß ihre Bestandteile vor der Verwendung getrennt verpackt vorliegen.

10. Geruchsbeseitigende Zusammensetzung nach einem der Ansprüche 1 bis 9, dadurch gekennzeichnet, daß jeder ihrer Bestandteile in Pulverform oder auch in Suspension oder in Lösung in Wasser oder auch wenigstens teilweise in adsorbiertem Zustand auf einem porösen und inerten Träger, wie Ton- oder Siliciumdioxidteilchen, vorliegt.

11. Verfahren zur Beseitigung des Geruchs von Gülle, insbesondere Gülle von Schweinen, dadurch gekennzeichnet, daß man eine Zusammensetzung nach einem der Ansprüche 1 bis 10 den zu behandelnden tierischen Exkrementen bei ihrer Lagerung oder ihrer Ausbringung durch Mischen zusetzt.

12. Verfahren nach Anspruch 11, dadurch gekennzeichnet, daß man die Mischung aus den einzelnen Bestandteilen der Zusammensetzung erst im Augenblick der Verwendung herstellt.

13. Verfahren nach Anspruch 11, dadurch gekennzeichnet, daß man erst im Augenblick der Verwendung den zu behandelnden tierischen Exkrementen mindestens einen Bestandteil der Zusammensetzung getrennt von den anderen Bestandteilen zusetzt.
